# EUROPEAN PATENT SPECIFICATION

(11) **EP 3 383 516 B1**
(45) Date of publication and mention of the grant of the patent: **25.12.2019**
(21) Application number: 16816482.0
(22) Date of filing: 22.11.2016
(51) Int. Cl.: B01D 15/36, B01J 41/05, B01J 41/20, C07C 51/47, C07C 53/122

(54) **CHROMATOGRAPHIC SEPARATION OF PROPIONIC ACID USING STRONG BASE ANION EXCHANGE RESIN**
CHROMATOGRAFISCHE TRENNUNG VON PROPIONSÄURE ANHAND EINES STARK BASISCHEN ANIONENAUSTAUSCHERHARZES
SÉPARATION CHROMATOGRAPHIQUE D'ACIDE PROPIONIQUE À L'AIDE DE RÉSINE ÉCHANGEUSE D'ANIONS À BASE FORTE

(30) Priority: 01.12.2015 US 201562261351 P
(43) Date of publication of application: 10.10.2018
(73) Proprietor: Dow Global Technologies, LLC, Midland, MI 48674 (US); Rohm and Haas Company, Philadelphia, PA 19106 (US)
(72) Inventor: PEASE, Stephen, Collegeville PA 19426 (US); GISCH, Daryl J., Midland MI 48674 (US); MARTIN, Collin H., Collegeville PA 19426 (US); MARTINS, Bianca F., 04794-000 Sao Paulo (BR)
(74) Representative: Beck Greener LLP
(86) International application number: PCT/US2016/063214
(87) International publication number: WO 2017/095685

(56) References cited:
- US-A- 2 748 163
- US-A- 5 132 456
- PENG WANG ET AL: "Novel in situ product removal technique for simultaneous production of propionic acid and vitamin B12 by expanded bed adsorption bioreactor", BIORESOURCE TECHNOLOGY, ELSEVIER BV, GB, vol. 104, 14 October 2011 (2011-10-14), pages 652-659, XP028351246, ISSN: 0960-8524, DOI: 10.1016/J.BIORTECH.2011.10.047 [retrieved on 2011-10-24]
- DATABASE WPI Week 198142 Thomson Scientific, London, GB; AN 1981-76930D XP002767107, & JP S56 113400 A (HITACHI LTD) 7 September 1981 (1981-09-07)
- PALATY Z ET AL: "Transport properties of propionic acid in anion-exchange membrane Neosepta-AFN", JOURNAL OF MEMBRANE SCIENCE, ELSEVIER BV, NL, vol. 349, no. 1-2, 1 March 2010 (2010-03-01), pages 90-96, XP026874416, ISSN: 0376-7388 [retrieved on 2009-12-11]

## Description

### FIELD

The invention relates the use of ion exchange resins to chromatographically separate propionic acid from a liquid feed mixture.

### INTRODUCTION

Propionic acid (also referred to as "propanoic acid") is currently used as food and feed additive and in grain preservation. Propionic acid may also be used as a precursor in polypropylene and vinyl propionate production. Traditionally, propionic acid has been produced by way of a petrochemical route. More recently, propionic acid can be made by fermentation processes. See for example: Zhu et al., Optimization and Scale-up of Propionic Acid Production by Propionic Acid-tolerant Propioni bacterium adicipropionici with Glycerol as the Carbon Source, Bioresource Technology 101 (2010), 8902-8906.

Traditional methods for purifying propionic acid include distillation and solvent extraction, (see US9024066). Unfortunately, distillation is energy intensive and creates undesired byproducts along with polymerization reactions that occur when heating organic acids to high temperatures. Solvent extraction uses low-boiling point solvents to remove propionic acid from aqueous solutions followed by evaporation of the solvent. This method is disadvantaged by the relative non-selectivity of solvent extraction, e.g. many other compounds will partition into the solvent phase and contaminate the product. Additionally, the best performing solvents are usually long chain alcohols that react with the acids in the media producing esters. Therefore, a reactive distillation to perform the hydrolysis of the esters and recover the acids is usually necessary in this process. Improved separation techniques are desired.

The separation of propionic acid with either strongly basic adsorbents or ion-exchange resins classed as weak to moderately basic anion exchangers, has also been proposed, see US 5,132,456.

### SUMMARY

The invention includes a method for chromatographically separating propionic acid from a liquid feed mixture including propionic acid and a carbohydrate by passing the liquid feed mixture through a bed including a strong base anion exchange resin. In a preferred embodiment, the anion exchange resin is a gel-type, Type I strong base resin. In another preferred embodiment, the liquid feed mixture is a fermentation process liquid including one or more organic acids, alcohols, inorganic salts and saccharides.

### BRIEF DESCRIPTION OF DRAWINGS

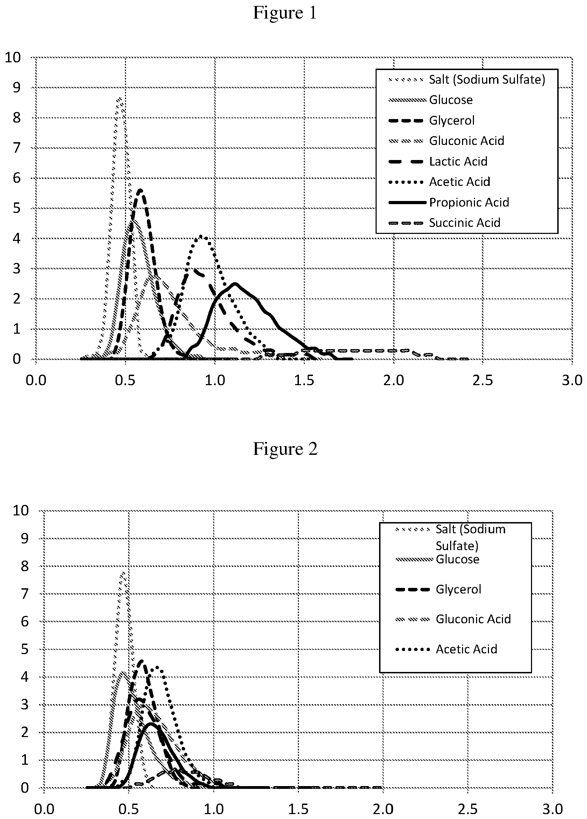

Figures 1 and 2 are plots of concentration (g/L) vs. column volume for selected compounds described in the Examples.

### DETAILED DESCRIPTION

The invention includes a method for chromatographically separating propionic acid from a liquid mixture (e.g. aqueous-based) including propionic acid and carbohydrate. In a preferred embodiment of the invention, the liquid feed mixture includes at least 1 g/L of propionic acid. Additionally, the liquid feed mixture may include one or more carbohydrates including saccharides (e.g. sucrose, glucose, fructose, xylose, mannose), derived from but not limited to sugarcane or beet sugar or corn syrup or from biomass hydrolysis, amino acids, alcohols (e.g. glycerol), amino acids, proteins, inorganic salts (e.g. salts of potassium sodium, calcium, magnesium, iron, and particularly sodium sulfate) and various organic acids, e.g. acetic acid, lactic acid, itaconic acid, succinic acid, maleic acid, citric acid, ascorbic acid, α-ketoglutaric acid, glycolic acid, gluconic acid, malic acid, tartaric acid and saccharic acid along with their corresponding salts. Representative liquid feed mixtures include those of used in fermentation processes, e.g. sugarcane, beet sugar, corn sugars, glycerol and biomass derived sugar fermentations. As with traditional chromatographic separations, the liquid feed mixture (mobile phase) passes through a bed or stratum of ion exchange resin (stationary phase). The set up and operation of the bed is not particularly limited, e.g. moving, simulated moving and stationary beds may be used. In a preferred embodiment, the chromatographic separation is a continuous process rather than batch.

The subject strong base anion exchange resin used as the stationary phase is preferably provided in bead form having a median diameter from 10 to 2000 microns, and more preferably from 100 to 1000 microns. The beads may have a Gaussian particle size distribution or may have a relatively uniform particle size distribution, i.e. "monodisperse" that is, at least 90 volume percent of the beads have a particle diameter from about 0.8 to about 1.2, and more preferably 0.85 to 1.15 times the volume average particle diameter. Monodisperse beads are preferred.

The subject anion exchange resins are preferably gel-type. The terms "microporous," "gellular," "gel" and "gel-type" are synonyms that describe copolymer resins having pore sizes less than about 20 Angstroms (Å). In distinction, macroporous resins have both mesopores of from about 20 Å to about 500 Å and macropores of greater than about 500 Å. Gel-type copolymer beads, as well as their preparation are described in US4256840 and US5244926. One preferred method is known in the art as a "seeded" polymerization, sometimes also referred to as batch or multi-batch (as generally described in EP 62088A1 and EP 179133A1); and continuous or semi-continuous staged polymerizations (as generally described in US 4,419,245; US 4,564,644; and US 5,244,926). A seeded polymerization process typically adds monomers in two or more increments. Each increment is followed by complete or substantial polymerization of the monomers therein before adding a subsequent increment. A seeded polymerization is advantageously conducted as a suspension polymerization wherein monomers or mixtures of monomers and seed particles are dispersed and polymerized within a continuous suspending medium. In such a process, staged polymerization is readily accomplished by forming an initial suspension of monomers, wholly or partially polymerizing the monomers to form seed particles, and subsequently adding remaining monomers in one or more increments. Each increment may be added at once or continuously. Due to the insolubility of the monomers in the suspending medium and their solubility within the seed particles, the monomers are imbibed by the seed particles and polymerized therein. Multi-staged polymerization techniques can vary in the amount and type of monomers employed for each stage as well as the polymerizing conditions employed.

The seed particles employed may be prepared by known suspension polymerization techniques. In general the seed particles may be prepared by forming a suspension of a first monomer mixture in an agitated, continuous suspending medium as described by F. Helfferich in Ion Exchange, (McGraw-Hill 1962) at pp. 35-36. The first monomer mixture comprises: 1) a first monovinylidene monomer, 2) a first crosslinking monomer, and 3) an effective amount of a first free-radical initiator. The suspending medium may contain one or more suspending agents commonly employed in the art. Polymerization is initiated by heating the suspension to a temperature of generally from about 50-90°C. The suspension is maintained at such temperature or optionally increased temperatures of about 90-150° C until reaching a desired degree of conversion of monomer to copolymer. Other suitable polymerization methods are described in US 4,444,961; US 4,623,706; US 4,666,673; and US 5,244,926 - each of which is incorporated herein in its entirety.

The monovinylidene aromatic monomers employed herein are well-known and reference is made to Polymer Processes, edited by Calvin E. Schildknecht, published in 1956 by Interscience Publishers, Inc., New York, Chapter III, "Polymerization in Suspension" at pp. 69-109. Table II (pp. 78-81) of Schildknecht lists diverse types of monomers which are suitable in practicing the present invention. Of the monomers listed, styrene and substituted styrene are preferred. The term "substituted styrene" includes substituents of either/or both the vinylidene group and phenyl group of styrene and include: vinyl naphthalene, alpha alkyl substituted styrene (e.g., alpha methyl styrene) alkylene-substituted styrenes (particularly monoalkyl-substituted styrenes such as vinyltoluene and ethylvinylbenzene) and halo-substituted styrenes, such as bromo or chlorostyrene and vinylbenzyl chloride. Additional monomers may be included along with the monovinylidene aromatic monomers, including monovinylidene non-styrenics such as: esters of α,β-ethylenically unsaturated carboxylic acids, particularly acrylic or methacrylic acid, methyl methacrylate, isobornyl- methacrylate, ethylacrylate, and butadiene, ethylene, propylene, acrylonitrile, and vinyl chloride; and mixtures of one or more of said monomers. Preferred monovinylidene monomers include styrene and substituted styrene such as ethylvinylbenzene. The term "monovinylidene monomer" is intended to include homogeneous monomer mixtures and mixtures of different types of monomers, e.g. styrene and isobornylmethacrylate. The seed polymer component preferably comprises a styrenic content greater than 50 molar percent, and more preferably greater than 75, and in some embodiments greater than 95 molar percent (based upon the total molar content). The term "styrenic content" refers to the quantity of monovinylidene monomer units of styrene and/or substituted styrene utilized to form the copolymer. "Substituted styrene" includes substituents of either/or both the vinylidene group and phenyl group of styrene as described above. In preferred embodiments, the first monomer mixture used to form the first polymer component (e.g. seed) comprises at least 75 molar percent, preferably at least 85 molar percent and in some embodiments at least 95 molar percent of styrene.

Examples of suitable crosslinking monomers (i.e., polyvinylidene compounds) include polyvinylidene aromatics such as divinylbenzene, divinyltoluene, divinylxylene, divinylnaphthalene, trivinylbenzene, divinyldiphenylsulfone, as well as diverse alkylene diacrylates and alkylene dimethacrylates. Preferred crosslinking monomers are divinylbenzene, trivinylbenzene, and ethylene glycol dimethacrylate. The terms "crosslinking agent," "crosslinker" and "crosslinking monomer" are used herein as synonyms and are intended to include both a single species of crosslinking agent along with combinations of different types of crosslinking agents. The proportion of crosslinking monomer in the copolymer seed particles is preferably sufficient to render the particles insoluble in subsequent polymerization steps (and also on conversion to an ion-exchange resin), yet still allow for adequate imbibition of an optional phase-separating diluent and monomers of the second monomer mixture. In some embodiments, no crosslinking monomer will be used. Generally, a suitable amount of crosslinking monomer in the seed particles is minor, i.e., desirably from about 0.01 to about 12 molar percent based on total moles of monomers in the first monomer mixture used to prepare the seed particles. In a preferred embodiment, the first polymer component (e.g. seed) is derived from polymerization of a first monomer mixture comprising at least 85 molar percent of styrene (or substituted styrene such as ethylvinylbenzene) and from 0.01 to about 10 molar percent of divinylbenzene.

Polymerization of the first monomer mixture may be conducted to a point short of substantially complete conversion of the monomers to copolymer or alternatively, to substantially complete conversion. If incomplete conversion is desired, the resulting partially polymerized seed particles advantageously contain a free-radical source therein capable of initiating further polymerization in subsequent polymerization stages. The term "free-radical source" refers to the presence of free-radicals, a residual amount of free-radical initiator or both, which is capable of inducing further polymerization of ethylenically unsaturated monomers. In such an embodiment of the invention, it is preferable that from about 20 to about 95 weight percent of the first monomer mixture, based on weight of the monomers therein, be converted to copolymer and more preferably from about 50 to about 90 weight percent. Due to the presence of the free radical source, the use of a free-radical initiator in a subsequent polymerization stage would be optional. For embodiments where conversion of the first monomer mixture is substantially complete, it may be necessary to use a free-radical initiator in subsequent polymerization stages.

The free-radical initiator may be any one or a combination of conventional initiators for generating free radicals in the polymerization of ethylenically unsaturated monomers. Representative initiators are UV radiation and chemical initiators, such as azo-compounds including azobisisobutyronitrile; and peroxygen compounds such as benzoyl peroxide, t-butylperoctoate, t-butylperbenzoate and isopropylpercarbonate. Other suitable initiators are mentioned in US 4192921, US 4246386 and US 4283499 - each of which is incorporated in its entirety. The free-radical initiators are employed in amounts sufficient to induce polymerization of the monomers in a particular monomer mixture. The amount will vary as those skilled in the art can appreciate and will depend generally on the type of initiators employed, as well as the type and proportion of monomers being polymerized. Generally, an amount of from about 0.02 to about 2 weight percent is adequate, based on total weight of the monomer mixture.

The first monomer mixture used to prepare the seed particles is advantageously suspended within an agitated suspending medium comprising a liquid that is substantially immiscible with the monomers, (e.g. preferably water). Generally, the suspending medium is employed in an amount from about 30 to about 70 and preferably from about 35 to about 50 weight percent based on total weight of the monomer mixture and suspending medium. Various suspending agents are conventionally employed to assist with maintaining a relatively uniform suspension of monomer droplets within the suspending medium. Illustrative suspending agents are gelatin, polyvinyl alcohol, magnesium hydroxide, hydroxyethylcellulose, methylhydroxyethyl cellulose methylcellulose and carboxymethyl methylcellulose. Other suitable suspending agents are disclosed in US4419245. The amount of suspending agent used can vary widely depending on the monomers and suspending agents employed. Latex inhibitors such as sodium dichromate may be used to minimize latex formation.

In the so-called "batch-seeded" process, seed particles comprising from about 10 to about 50 weight percent of the copolymer are preferably suspended within a continuous suspending medium. A second monomer mixture containing a free radical initiator is then added to the suspended seed particles, imbibed thereby, and then polymerized. Although less preferred, the seed particles can be imbibed with the second monomer mixture prior to being suspended in the continuous suspending medium. The second monomer mixture may be added in one amount or in stages. The second monomer mixture is preferably imbibed by the seed particles under conditions such that substantially no polymerization occurs until the mixture is substantially fully imbibed by the seed particles. The time required to substantially imbibe the monomers will vary depending on the copolymer seed composition and the monomers imbibed therein. However, the extent of imbibition can generally be determined by microscopic examination of the seed particles, or suspending media, seed particles and monomer droplets. The second monomer mixture desirably contains from about 0.5 to about 25 molar percent, preferably from about 2 to about 17 molar percent and more preferably 2.5 to about 8.5 molar percent of crosslinking monomer based on total weight of monomers in the second monomer mixture with the balance comprising a monovinylidene monomer; wherein the selection of crosslinking monomer and monovinylidene monomer are the same as those described above with reference to the preparation of the first monomer mixture, (i.e. seed preparation). As with the seed preparation, the preferred monovinylidene monomer includes styrene and/or a substituted styrene. In a preferred embodiment, the second polymer component (i.e. second monomer mixture, or "imbibed" polymer component) has a styrenic content greater than 50 molar percent, and more preferably at least 75 molar percent (based upon the total molar content of the second monomer mixture). In a preferred embodiment, the second polymer component is derived from polymerization of a second monomer mixture comprising at least 75 molar percent of styrene (and/or substituted styrene such as ethylvinylbenzene) and from about 1 to 20 molar percent divinylbenzene.

In an in-situ batch-seeded process, seed particles comprising from about 10 to about 80 weight percent of the copolymer product are initially formed by suspension polymerization of the first monomer mixture. The seed particles can have a free-radical source therein as previously described, which is capable of initiating further polymerization. Optionally, a polymerization initiator can be added with the second monomer mixture where the seed particles do not contain an adequate free radical source or where additional initiator is desired. In this embodiment, seed preparation and subsequent polymerization stages are conducted in-situ within a single reactor. A second monomer mixture is then added to the suspended seed particles, imbibed thereby, and polymerized. The second monomer mixture may be added under polymerizing conditions, but alternatively may be added to the suspending medium under conditions such that substantially no polymerization occurs until the mixture is substantially fully imbibed by the seed particles. The composition of the second monomer mixture preferably corresponds to the description previously given for the batch-seeded embodiment.

The copolymer product is preferably chloromethylated and subsequently aminated. The specific means and conditions for chloromethylating the copolymers are not particularly limited and many applicable techniques are documented in the literature, as illustrated by: G. Jones, "Chloromethylation of Polystyrene," Industrial and Engineering Chemistry, Vol. 44, No. 1, pgs. 2686-2692, (Nov 1952), along with US 2008/0289949 and US 6756462 - both of which are incorporated herein in their entirety. Chloromethylation is typically conducted by combining the polymer with a chloromethylation reagent in the presence of a catalyst at a temperature of from about 15 to 100°C, preferably 35 to 70°C for about 1 to 8 hours. A preferred chloromethylation reagent is chloromethyl methyl ether (CMME); however, other reagents may be used including CMME-forming reactants such as the combination of formaldehyde, methanol and hydrogen chloride or chlorosulfonic acid (as described in US 2004/0256597), or hydrogen chloride with methylated formalin. The chloromethylating reagent is typically combined with the polymer in an amount of from about 0.5 to 20, preferably about 1.5 to 8 mole of CMME per mole of polymer. While less preferred, other chloromethylation reagents may be used including but not limited to: bis-chloromethyl ether (BCME), BCME-forming reactants such as formaldehyde and hydrogen chloride, and long chain alkyl chloromethyl ethers as described in US 4568700.

Catalyst useful for conducting chloromethylation reactions are well known and are often referred to in the art as "Lewis acid" or "Friedel-Crafts" catalyst. Non-limiting examples include: zinc chloride, zinc oxide, ferric chloride, ferric oxide, tin chloride, tin oxide, titanium chloride, zirconium chloride, aluminum chloride and sulfuric acid along with combinations thereof. Halogens other than chloride may also be used in the preceding examples. A preferred catalyst is ferric chloride. The catalyst is typically used in an amount corresponding to about 0.01 to 0.2, preferably from about 0.02 to 0.1 mole catalyst per mole of polymer repeating unit. Catalyst may be used in combination with optional catalyst adjuncts such as calcium chloride and activating agents such as silicon tetrachloride. More than one catalyst may be used to achieve the desired chloromethylation reaction profile.

Solvents and/or swelling agents may also be used in the chloromethylation reaction. Examples of suitable solvents including but are not limited to one or more of: an aliphatic hydrocarbon halides such as ethylene dichloride, dichloropropane, dichloromethane, chloroform, diethyl ether, dipropyl ether, dibutyl ether and diisoamyl ether. When CMME is used as the chloromethylation agent, such solvents and/or swelling agents are often not necessary.

The chloromethylated vinyl aromatic polymer is reacted with an amine to form an ion exchange resin including functional amine groups. The amination is preferably conducted by combining a tertiary amine and a vinyl aromatic polymer comprising benzyl chloride groups within an alcohol-based solvent to form a reaction mixture. A preferred tertiary amine is represented by the Formula 1. 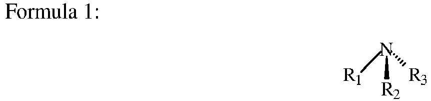 wherein R₁, R₂ and R₃ are each independently selected from: alkyl and alkoxy groups each having from 1 to 6 carbon atoms but preferably 1 to 2 carbon atoms. Each alkyl or alkoxy group (R₁, R₂ and R₃) may independently be: straight (e.g. ethyl, propyl, butyl, pentyl, etc.) or branched (e.g. isopropyl, isobutyl, etc.), and may be unsubstituted or substituted (e.g. substituted with such groups as a hydroxyl). In one series of embodiments, the three alkyl groups (R₁, R₂ and R₃) are independently selected from unsubstituted alkyl groups which may be straight or branched. In other embodiments, "mixed species" of the subject tertiary amines may be used. Representative amines include: trimethylamine, dimethylaminoethanol, triethylamine, tripropylamine and tributylamine. A preferred amine is trimethyl amine. A reaction product of the subject amination, i.e. a vinyl aromatic polymer including a quaternary ammonium functionality including a nitrogen atom bonded to a benzyl carbon of the polymer and three alkyl or alkoxy groups. By way of illustration, Formula 2 provides a structural formula of a repeating unit of vinyl aromatic polymer including quaternary ammonium functionality. 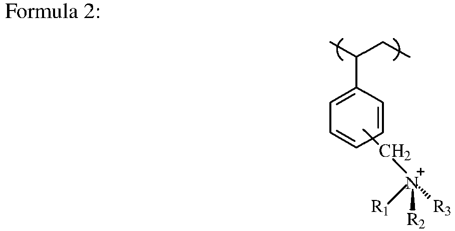 wherein the benzyl carbon is located at the meta, ortho or para position (typically including a combination of species but with predominantly para substitution) of the aromatic ring; and wherein R₁, R₂ and R₃ are the same as previously described with respect to Formula 1. Representative amination reactions are described in: US5134169, US6756462, US 6924317, US8273799 and US 2004/0256597.

Representative strong base anion exchange resins include DOWEX™ 1x4 and DOWEX™ MARATHON 11, both commercially available from The Dow Chemical Company.

### EXAMPLES

Individual compounds (identified in the Figures) were injected into a packed resin bed (91 cm long x 2.5 cm diameter) of a strong base anion (Type I) gel-type resin including a crosslinked copolymer matrix derived from reaction mixture including styrene and divinylbenzene in sulfate form (Figure 1) and for comparison, a weak base anion resin, AMBERLITE™ CR5550 (Figure 2), at a concentration of 20 wt% or the maximum solubility of the compound in 2 g/L sulfuric acid at room temperature, whichever was lower. The injection volume was 0.05 column volumes (26.3 mL). The mobile phase was 2 g/L sulfuric acid and the column flowrate and temperature were 3.0 column volumes/hr (26.2 mL/min) and 53°C respectively. Elution fractions (8 mL each) were collected and analyzed by a Reichert AR200 hand-held refractometer to determine compound concentration in each fraction. Compound standards of known concentration were used to convert refractometer signal into concentration in g/L. The results of this analysis for each component are overlaid in the Figures.

## Claims

1. A method for chromatographically separating propionic acid from a liquid feed mixture comprising propionic acid and a carbohydrate by passing the liquid feed mixture through a bed comprising a strong base anion exchange resin.

2. The method of claim 1 wherein the anion exchange resin comprises a crosslinked copolymer matrix derived from reaction of at least one monovinyl monomer and a polyvinyl aromatic crosslinking monomer.

3. The method of claim 2 wherein the monovinyl monomer comprises styrene and the polyvinyl aromatic crosslinking monomer comprises divinylbenzene.

4. The method of claim 1 wherein the anion exchange resin is a gel-type resin.

5. The method of claim 1 wherein the liquid feed mixture further comprises one or more of: an organic acid other than propionic acid, an alcohol and an inorganic salt.

6. The method of claim 1 wherein the liquid feed mixture comprises a fermentation broth derived from at least one of: sugarcane, beet sugar, corn sugars, glycerol and biomass derived sugar fermentation.

## Patentansprüche

1. Verfahren zum chromatographischen Abtrennen von Propansäure aus einem Propansäure und ein Kohlenhydrat umfassenden flüssigen Speisegemisch, indem das flüssige Speisegemisch durch ein Bett geleitet wird, das ein stark basisches Anionenaustauscherharz umfasst.

2. Verfahren nach Anspruch 1, wobei das Anionenaustauscherharz eine vernetzte Copolymermatrix umfasst, die aus der Reaktion von mindestens einem Monovinylmonomer und einem polyvinylaromatischen vernetzenden Monomer abgeleitet ist.

3. Verfahren nach Anspruch 2, wobei das Monovinylmonomer Styrol umfasst und das polyvinylaromatische vernetzende Monomer Divinylbenzol umfasst.

4. Verfahren nach Anspruch 1, wobei das Anionenaustauscherharz ein Harz vom Gel-Typ ist.

5. Verfahren nach Anspruch 1, wobei das flüssige Speisegemisch ferner eine oder mehrere der folgenden Substanzen umfasst: eine andere organische Säure als Propansäure, einen Alkohol und ein anorganisches Salz.

6. Verfahren nach Anspruch 1, wobei das flüssige Speisegemisch eine Fermentationsbrühe umfasst, die abgeleitet ist von mindestens einer der folgenden Substanzen: Zuckerrohr, Rübenzucker, Maiszucker, Glycerin und aus Zuckerfermentation abgeleitete Biomasse.

## Revendications

1. Méthode pour une séparation chromatographique d'acide propionique d'un mélange d'alimentation liquide comprenant de l'acide propionique et un hydrate de carbone en faisant passer le mélange d'alimentation liquide à travers un lit comprenant une résine échangeuse d'anions à base forte.

2. Méthode selon la revendication 1, dans laquelle la résine échangeuse d'anions comprend une matrice de copolymère réticulé dérivée de la réaction d'au moins un monomère de monovinyle et d'un monomère de réticulation aromatique de polyvinyle.

3. Méthode selon la revendication 2, dans laquelle le monomère de monovinyle comprend du styrène et le monomère de réticulation aromatique de polyvinyle comprend du divinylbenzène.

4. Méthode selon la revendication 1, dans laquelle la résine échangeuse d'anions est une résine de type gel.

5. Méthode selon la revendication 1, dans laquelle le mélange d'alimentation liquide comprend en outre un ou plusieurs parmi: un acide organique autre que l'acide propionique, un alcool et un sel inorganique.

6. Méthode selon la revendication 1, dans laquelle le mélange d'alimentation liquide comprend un bouillon de fermentation dérivé d'au moins un parmi: canne à sucre, sucre de betterave, sucres de maïs, glycérol et une biomasse dérivée de la fermentation de sucre.
